# EUROPÄISCHE PATENTSCHRIFT

(11) **EP 0 600 958 B1**
(45) Veröffentlichungstag und Bekanntmachung des Hinweises auf die Patenterteilung: **20.03.1996**
(21) Anmeldenummer: 92917513.1
(22) Anmeldetag: 20.08.1992
(51) Int. Cl.: C07C 67/31, C07C 69/708, C07C 69/675, C07C 29/10, C07C 41/03, C07C 31/20, C07C 43/13

(54) **Epoxidringöffnungsprodukte und Verfahren zur Herstellung**
Epoxy-ring opening products and method of producing
Produits d'ouverture du cycle epoxy et procédé de fabrication

(30) Priorität: 29.08.1991 DE 4128649
(43) Veröffentlichungstag der Anmeldung: 15.06.1994
(73) Patentinhaber: Henkel Kommanditgesellschaft auf Aktien, D-40191 Düsseldorf (DE)
(72) Erfinder: DAUTE, Peter, D-4300 Essen 1 (DE); KLEIN, Johann, D-4000 Düsseldorf 1 (DE); GRÜTZMACHER, Roland, D-5603 Wülfrath (DE); HÖFER, Rainer, D-4000 Düsseldorf 30 (DE)
(86) Internationale Anmeldenummer: EP9201905
(87) Internationale Veröffentlichungsnummer: WO9305008

(56) Entgegenhaltungen:
- EP-A- 0 437 001
- WO-A-91/11424
- CH-A- 484 861

## Beschreibung

Die Erfindung betrifft Epoxidringöffnungsprodukte, erhältlich durch Umsetzung von Epoxidverbindungen mit Nucleophilen in Gegenwart von Lithiumsalzen und gegebenenfalls Umesterung der als Zwischenprodukte gebildeten Hydroxyverbindungen mit Fettsäureglyceridestern, ein Verfahren zu ihrer Herstellung sowie ihre Verwendung zur Herstellung von Polymeren.

### Stand der Technik

Zur Herstellung von Polyurethanschäumen werden Diisocyanate mit Stoffen umgesetzt, die mindestens zwei freie Hydroxylgruppen aufweisen. Hydroxyverbindungen, die sich für die Herstellung derartiger Kunststoffe besonders eignen, werden üblicherweise durch Ringöffnung von Epoxidverbindungen mit Nucleophilen, beispielsweise Alkoholen, in Gegenwart alkalischer oder saurer Katalysatoren hergestellt [**Fette, Seifen, Anstrichmitt., 89, 147 (1987)**].

In der US-amerikanische Patentschrift **US 4 057 589** ist beispielsweise ein Verfahren zur Herstellung von Tetrolen beschrieben, bei dem man ungesättigte Diole mit Peressigsäure umsetzt und die gebildeten Epoxide anschließend bei Temperaturen von mindestens 120°C hydrolysiert.

Gegenstand der Europäischen Patentanmeldung **EP 0 127 810 A1** ist die schwefelsäure-katalysierte Ringöffnung von Epoxiden ungesättigter Fettsäureester mit Alkoholen sowie deren nachfolgende Verseifung.

In der Deutschen Offenlegungsschrift **DE 32 46 612 A1** wird ein Verfahren zur Herstellung modifizierter Triglyceride vorgeschlagen, bei dem man epoxidierte Fette oder Öle in Gegenwart von Schwefelsäure, Phosphorsäure oder Sulfonsäuren mit ein- oder mehrwertigen Alkoholen umsetzt.

In der Europäischen Patentschrift **EP 0 257 332 B1** wird schließlich ein Verfahren zur kontinuierlichen Herstellung von 1,2 Diolen beschrieben, bei dem man Epoxide in Gegenwart von sauren Katalysatoren einer Druckspaltung mit Wasser unterwirft.

Die **EP 0 437 001 A1** beschreibt die Umsetzung epoxidierter Fettsäureester mit Acrylsäuren unter LiOH-Katalyse.

Die **WO 91/11424** beschreibt die Synthese alkoxylierter Verbindungen, die Reaktionsprodukte von epodidierten Carbonsäurederivaten und alkylenoxiden in Gegenwart eines mono- oder polyfunktionellen Alkohols darstellen.

Die **CH-A-484 861** beschreibt die Synthese von Hydroxyalkoxypropionsäuren durch Ringöffnung eines epoxidierten Na-, K- oder Li-Salzes der Propionsäure mit einem Alkanoat.

Die Verfahren des Stands der Technik weisen übereinstimmend den Nachteil auf, daß die sauren oder alkalischen Katalysatoren nach der Reaktion neutralisiert werden müssen. Die dabei anfallenden Salze müssen mit hohem technischen Aufwand abgetrennt werden, da sie andernfalls zu Austrübungen in den Produkten oder einer unerwünscht heftigen Reaktion zwischen Polyol und Diisocyanat führen können.

Die Aufgabe der Erfindung bestand somit darin, ein Verfahren zur Herstellung von Epoxidringöffnungsprodukten zu entwikkeln, das frei von den geschilderten Nachteilen ist.

### Beschreibung der Erfindung

Gegenstand der Erfindung ist ein Verfahren zur Herstellung von Epoxidringöffnungsprodukten, das sich dadurch auszeichnet, daß man
a) Epoxidverbindungen mit Nucleophilen mit Ausnahme von Carbonsäuren in Gegenwart von 0,001 bis 0,01 Gew.-% - bezogen auf die Einsatzstoffe - an Lithiumsalzen umsetzt und
b) die entstehenden Hydroxyverbindungen gegebenenfalls mit Fettsäureglyceridestern einer Umesterung unterwirft.

Überraschenderweise wurde gefunden, daß die Ringöffnung von Epoxiden bereits in Gegenwart kleinster Mengen Lithiumhydroxid und/oder Lithiumfettsäuresalzen rasch und praktisch quantitativ abläuft. Die in den Epoxidringöffnungsprodukten noch enthaltenen Lithiummengen sind ferner ausreichend, um gegebenenfalls eine Umesterung mit Fettsäureglyceridestern zu katalysieren. Ein weiterer Vorteil des erfindungsgemäßen Verfahrens ist darin zu sehen, daß auf eine Neutralisation und/oder Abtrennung der Lithiumsalze verzichtet werden kann, da die geringen Einsatzmengen weder die Eigenschaften der Produkte, noch deren Verhalten in der Weiterverarbeitung negativ beeinflussen.

Epoxidverbindungen stellen bekannte Stoffe dar und können nach an sich bekannten Verfahren durch Epoxidation ungesättigter Einsatzstoffe erhalten werden. Beispiele hierzu sind die Umsetzung von Olefinen mit Peressigsäure in Anwesenheit saurer Katalysatoren [**DE 857 364**] oder mit in-situ aus Ameisensäure und Wasserstoffperoxid gebildeter Perameisensäure [**US 2 485 160**]. Für die Durchführung des erfindungsgemäßen Verfahrens ist Voraussetzung, daß in den Epoxidverbindungen ein substantieller Anteil, beispielsweise 2 bis 40, vorzugsweise 4 bis 8,5 Gew.-% Epoxidsauerstoff vorhanden ist. Dies schließt mit ein, daß im Sinne des erfindungsgemäßen Verfahrens nicht nur vollständig, sondern auch partiell epoxidierte Stoffe eingesetzt werden können.

Unter **Epoxidverbindungen** sind zu verstehen
a1) **Ethylenoxid**, **Propylenoxid** und/oder **Butylenoxid**,
a2) **Epoxide von Olefinen** der Formel **(I)**,

   R¹-CH=CH-R (I)

   in der R¹ für einen linearen oder verzweigten aliphatischen Kohlenwasserstoffrest mit 1 bis 18 Kohlenstoffatomen und R für Wasserstoff oder einen linearen oder verzweigten Kohlenwasserstoffrest mit 1 bis 8 Kohlenstoffatomen steht. Typische Beispiele sind die Epoxide von Octen-1, Decen-1, Dodecen-1, Tetradecen-1, Octadecen-1 oder Octadecen-9. Bevorzugt sind Epoxide von Olefinen der Formel (I), in der die Summe von R¹ und R für Zahlen von 8 bis 16 steht.
a3) **Epoxide von Estern** der Formel **(II)**,

   R³CO-OR⁴ (II)

   in der R³CO für einen aliphatischen Acylrest mit 16 bis 24 Kohlenstoffatomen und 1 bis 5 Doppelbindungen und R⁴ für einen linearen oder verzweigten Alkylrest mit 1 bis 4 Kohlenstoffatomen steht. Typische Beispiele sind die Epoxide von Palmitoleylsäuremethylester, Ölsäuremethylester, Elaidinsäuremethylester, Petroselinsäuremethylester, Linolsäuremethylester oder Erucasäuremethylester. Bevorzugt sind Epoxide von Estern der Formel (II), in der R³CO für einen aliphatischen Kohlenwasserstoffrest mit 18 bis 22 Kohlenstoffatomen und 1 oder 2 Doppelbindungen und R⁴ für eine Methylgruppe steht.
a4) **Epoxide von Estern** der Formel **(III)**,

   R⁵CO-OR⁶ (III)

   in der R⁵CO für einen aliphatischen Acylrest mit 1 bis 24 Kohlenstoffatomen und 0 oder 1 bis 5 Doppelbindungen und R⁶ für einen linearen oder verzweigten aliphatischen Kohlenwasserstoffrest mit 16 bis 24 Kohlenstoffatomen und 1 bis 5 Doppelbindungen steht. Typische Beispiele sind Epoxide von Essigsäureoleylester, Ölsäureoleylester oder Erucasäureoleylester. Bevorzugt sind Epoxide von Estern der Formel (III), in der R⁵CO für einen aliphatischen Acylrest mit 18 bis 22 Kohlenstoffatomen und 1 oder 2 Doppelbindungen und R⁶ für einen aliphatischen Kohlenwasserstoffrest mit 16 bis 22 Kohlenstoffatomen und 1 oder 2 Doppelbindungen steht.
a5) **Epoxide von Fettsäureglyceridestern** der Formel **(IV)**, 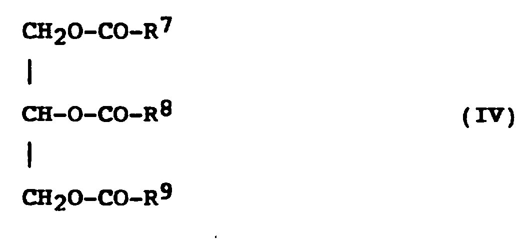 in der R⁷CO für einen linearen oder verzweigten aliphatischen Acylrest mit 16 bis 24 Kohlenstoffatomen und 1 bis 5 Doppelbindungen und R⁸CO und R⁹CO unabhängig voneinander für einen linearen oder verzweigten aliphatischen Acylrest mit 6 bis 22 Kohlenstoffatomen und 0 oder 1 bis 5 Doppelbindungen und deren Gemische steht. Typische Beispiele sind Epoxide von Erdnußöl, Korianderöl, Baumwollsaatöl, Olivenöl, Leinöl, Rindertalg, Fischöl oder insbesondere Sojaöl. Bevorzugt ist der Einsatz von Epoxiden von Glycerinfettsäureestern der Formel **(IV)**, in der R⁷CO, R⁸CO und R⁹CO unabhängig voneinander für aliphatische Acylreste mit 18 bis 22 Kohlenstoffatomen und überwiegend 1 oder 2 Doppelbindungen stehen.

Als **Nucleophile**, die für die Ringöffnung der Epoxidverbindungen benötigt werden, kommen die folgenden Verbindungen in Betracht:
b1) **Wasser**
b2) **Alkohole** der Formel **(V)**,

   R¹⁰OH (V)

   in der R¹⁰ für lineare oder verzweigte aliphatische Kohlenwasserstoffreste mit 1 bis 22 Kohlenstoffatomen und 0 oder 1 bis 3 Doppelbindungen steht. Typische Beispiele sind Methanol, Ethanol, Propanol-1, Propanol-2, n-Butanol, Pentanol, Hexanol, Octanol, Decanol, Laurylalkohol, Myristylalkohol, Cetylalkohol, Stearylalkohol, Oleylalkohol, Behenylalkohol oder Erucylalkohol. Bevorzugt ist der Einsatz von Methanol und Ethanol.
b3) **Mehrwertige Alkohole**, ausgewählt aus der Gruppe, die von Ethylenglycol, Diethylenglycol, Polyethylenglycole im Molgewichtsbereich 300 bis 1500, Propandiol-1,2, Propandiol-1,3, Glycerin, Oligoglycerine mit Kondensationsgraden von durchschnittlich 2 bis 10, Trimethylolpropan, Pentaerythrit, Sorbitol und Sorbitan gebildet wird.
b4) **Fettalkoholpolyglycolether** der Formel **(VI)**, 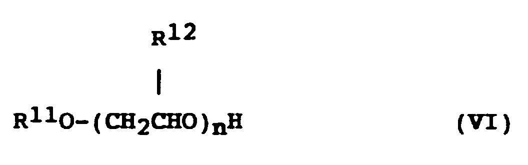 in der R¹¹ für lineare oder verzweigte aliphatische Kohlenwasserstoffreste mit 6 bis 22 Kohlenstoffatomen und 0 oder 1 bis 3 Doppelbindungen, R¹ für Wasserstoff oder eine Methylgruppe und n für Zahlen von 1 bis 30 steht. Typische Beispiele sind Anlagerungsprodukte von durchschnittlich 1 bis 30 Mol Ethylen- und/oder Propylenoxid an jeweils 1 Mol Hexanol, Octanol, Decanol, Lauryalkohol, Myristylalkohol, Cetylalkohol, Stearylalkohol, Oleylalkohol, Behenylalkohol oder Erucylalkohol. Bevorzugt ist der Einsatz von Fettalkoholpolyglycolethern der Formel **(VI)**, in der R¹¹ für Alkylreste mit 6 bis 18 Kohlenstoffatomen, R¹ für Wasserstoff und n für Zahlen von 1 bis 10 steht.

Die Epoxidverbindungen und die Nucleophile können in molaren Verhältnissen von 1 : 10 bis 10 : 1, vorzugsweise 1 : 3 bis 3 : 1 eingesetzt werden.

Als **Lithiumsalze** kommen neben Lithiumhydroxid auch Lithiumseifen, also Salze des Lithiums mit gegebenenfalls hydroxyfunktionalisierten Fettsäuren mit 6 bis 22 Kohlenstoffatomen und 0, 1, 2 oder 3 Doppelbindungen in Betracht. Typische Beispiele sind die Lithiumsalze der Capronsäure, Caprylsäure, Caprinsäure Laurinsäure, Myristinsäure, Palmitinsäure, Palmitoleinsäure, Stearinsäure, Ölsäure, Elaidinsäure, Petroselinsäure, Linolsäure, Linolensäure, 12-Hydroxystearinsäure, Ricinolsäure, Arachinsäure, Gadoleinsäure, Behensäure oder Erucasäure. Die Lithiumsalze können in Form wäßriger oder alkoholischer Lösung zum Einsatz gelangen. Wegen ihrer leichten Dosierbarkeit werden vorzugsweise die ohnehin flüssigen Lithiumsalze ungesättigter Fettsäuren eingesetzt. Die Lithiumseifen können der Reaktionsmischung direkt zugesetzt werden; sie können sich jedoch auch in situ bilden, beispielsweise aus Lithiumhydroxid und einem Fettsäureester.

Die Einsatzmenge der Lithiumsalze kann 0,001 bis 0,1, vorzugsweise 0,002 bis 0,05 und insbesondere 0,005 bis 0,01 Gew.-% - bezogen auf die Einsatzstoffe - betragen. Da Lithiumionen mit den in Gläsern enthaltenen Alkaliionen austauschen können, was zu einer Verringerung der Konzentration an Lithiumionen führen kann, empfiehlt es sich, das Verfahren in Geräten aus Stahl oder ähnlich inerten Materialien durchzuführen.

Die Ringöffnung kann in an sich bekannter Weise erfolgen, wobei es sich als vorteilhaft erwiesen hat, die Reaktion bei der Siedetemperatur des eingesetzten Nucleophils bzw. im Temperaturbereich von 100 bis 250°C durchzuführen. Dabei ist es nicht zwingend erforderlich, daß die Ringöffnung vollständig abläuft. Es ist vielmehr ebenso möglich, Epoxidringöffnungsprodukte herzustellen, die noch über einen definierten Restgehalt an Epoxidsauerstoff, beispielsweise 1 bis 3 Gew.-%, verfügen.

Als **Fettsäureglyceridester**, mit denen die Ringöffnungsprodukte gegebenenfalls umgeestert werden können, kommen Triglyceride der Formel **(VII)** in Betracht, 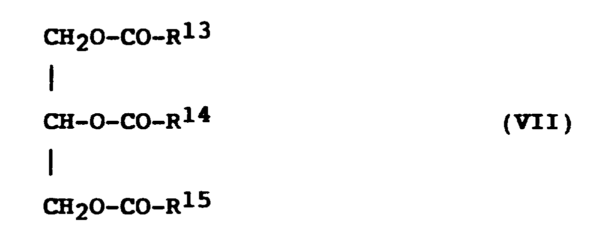 in der R¹³CO für einen linearen oder verzweigten, gegebenenfalls hydroxy- und/oder alkoxysubstituierten aliphatischen Acylrest mit 16 bis 24 Kohlenstoffatomen und 1 bis 5 Doppelbindungen und R¹⁴CO und R¹⁵CO unabhängig voneinander für einen linearen oder verzweigten aliphatischen Acylrest mit 6 bis 24 Kohlenstoffatomen und 0 oder 1 bis 5 Doppelbindungen steht. Typische Beispiele sind natürliche Fettsäureglyceridester pflanzlicher oder tierischer Herkunft auf Basis von Rüböl, Ricinusöl, Palmöl, Sojaöl oder Kokosöl sowie chemisch modifizierte Triglyceride vom Typ der Sojapolyole, die man durch Epoxidation von Sojaöl und nachfolgende Öffnung der Oxiranringe mit geeigneten Nucleophilen, beispielsweise Alkoholen, erhält.

Das molare Verhältnis zwischen Epoxiden und Fettsäureglyceridestern kann ebenfalls 10 : 1 bis 1 : 10, vorzugsweise 3 : 1 bis 1 : 3 betragen. Die Umesterung kann in an sich bekannter Weise im Temperaturbereich von 100 bis 250°C durchgeführt werden. Wie schon zuvor ausgeführt, ist die im Ringöffnungsprodukt verbliebene Lithiumkonzentration ausreichend, auch die Umesterung zu katalysieren. Der Zusatz weiteren Katalysators ist somit nicht erforderlich. Die Umesterung kann dabei vollständig oder partiell, beispielsweise zu 10 bis 90, insbesondere 20 bis 70 Gew.-% - bezogen auf den Fettsäureglyceridester - erfolgen.

In einer bevorzugten Ausführungsform der Erfindung können Ringöffnung und Umesterung nicht nacheinander, sondern in einem Schritt durchgeführt werden. Sofern noch im Produkt enthaltenes nichtumgesetztes Nucleophil die anwendungstechnischen Eigenschaften der Epoxidringöffnungsprodukte nachteilig beeinflußt oder in der Weiterverarbeitung der Stoffe störend wirkt, können diese beispielsweise destillativ abgetrennt werden. Dabei ist es unkritisch, ob sich die Destillation an die Ringöffnung oder die Umesterung anschließt.

Ein weiterer Gegenstand der Erfindung betrifft Epoxidringöffnungsprodukte, dadurch erhältlich, daß man
a) Epoxidverbindungen mit Nucleophilen mit Ausnahme von Carbonsäuren in Gegenwart 0,001 bis 0,01 Gew.-% - bezogen auf die Einsatzstoffe - von Lithiumsalzen umsetzt und
b) die entstehenden Hydroxyverbindungen gegebenenfalls mit Fettsäureglyceridestern einer Umesterung unterwirft.

### Gewerbliche Anwendbarkeit

Die nach dem erfindungsgemäßen Verfahren hergestellten Epoxidringöffnungsprodukte eignen sich als Rohstoffe zur Herstellung von Polymeren. Sie können beispielsweise in Alkydharze sowohl über die Hydroxylfunktionen als auch über noch im Molekül befindliche Epoxidgruppen einkondensiert werden und stellen mehrfunktionelle Polykondensationsbausteine dar, wie sie insbesondere für die Entwicklung von Polyurethanschäumen von Wichtigkeit sind.

Ein weiterer Gegenstand der Erfindung betrifft daher die Verwendung der erfindungsgemäßen Epoxidringöffnungsprodukte zur Herstellung von Polymeren, in denen sie zu 1 bis 90, vorzugsweise 10 bis 70 Gew.-% - bezogen auf die Polymeren - enthalten sein können.

Die folgenden Beispiele sollen den Gegenstand der Erfindung näher erläutern, ohne ihn darauf einzuschränken.

### Beispiele

### Beispiel 1:

In einem 2-l-Dreihalskolben mit Rührer wurde ein Gemisch aus

| | |
|---|---|
| 260 g | Sojaölepoxid, Epoxidsauerstoffgehalt 6,68 Gew.-%, entsprechend 1,08 Mol Epoxid, |
| 741 g (0,83 Mol) | Rüböl neuer Züchtung, Ölsäuregehalt > 80 Gew.-% und |
| 299 g (3,25 Mol) | Glycerin |

vorgelegt und unter Rühren mit 0,13 g (0,0054 Mol) Lithiumhydroxid, entsprechend 0,01 Gew.-% - bezogen auf die Einsatzstoffe - versetzt. Die Reaktionsmischung wurde auf 220°C erhitzt. Der Verlauf der Ringöffnungsreaktion wurde über die Änderung der Konzentration an Epoxidsauerstoff in der Reaktionsmischung verfolgt. Die Ergebnisse sind in Tab.l zusammengefaßt.

### Beispiel 2:

Analog Beispiel 1 wurden 260 g Sojölepoxid, 741 g Rüböl und 299 g Glycerin bei 240°C in Gegenwart von 0,03 g Lithiumhydroxid, entsprechend 0,002 Gew.-% - bezogen auf die Einsatzstoffe - umgesetzt. Der Verlauf der Ringöffnungsreaktion wurde über die Änderung der Konzentration an Epoxidsauerstoff in der Reaktionsmischung verfolgt. Die Ergebnisse sind in Tab.1 zusammengefaßt.

### Beispiel 3:

Analog Beispiel 1 wurden 390 g Sojölepoxid, entsprechend 1,6 Mol Epoxid, 611 g (0,7 Mol) Rüböl neuer Züchtung und 299 g Glycerin bei 220 bis 240°C in Gegenwart von 0,13 g Lithiumhydroxid, entsprechend 0,01 Gew.-% - bezogen auf die Einsatzstoffe - umgesetzt. Der Verlauf der Ringöffnungsreaktion wurde über die Änderung der Konzentration an Epoxidsauerstoff in der Reaktionsmischung verfolgt. Die Ergebnisse sind in Tab.l zusammengefaßt.

### Beispiel 4:

Analog Beispiel 1 wurden 260 g Epoxystearinsäuremethylester, 910 g (1 Mol) Rüböl neuer Züchtung und 130 g (1,4 Mol) Glycerin bei 220 bis 240°C in Gegenwart von 0,13 g Lithiumhydroxid, entsprechend 0,01 Gew.-% - bezogen auf die Einsatzstoffe - umgesetzt. Der Verlauf der Ringöffnungsreaktion wurde über die Änderung der Konzentration an Epoxidsauerstoff in der Reaktionsmischung verfolgt. Die Ergebnisse sind in Tab.1 zusammengefaßt.

### Beispiel 5:

Analog Beispiel 1 wurden 520 g Epoxystearinsäuremethylester, 520 g (0,6 Mol) Rüböl neuer Züchtung und 260 g (2,8 Mol) Glycerin bei 220 bis 240°C in Gegenwart von 0,13 g Lithiumhydroxid, entsprechend 0,01 Gew.-% - bezogen auf die Einsatzstoffe - umgesetzt. Der Verlauf der Ringöffnungsreaktion wurde über die Änderung der Konzentration an Epoxidsauerstoff in der Reaktionsmischung verfolgt. Die Ergebnisse sind in Tab.1 zusammengefaßt.

### Vergleichsbeispiel 1:

Analog Beispiel 1 wurden 260 g Sojölepoxid, 741 g Rüböl und 299 g Glycerin bei 240°C in Gegenwart von 0,26 g Kaliumhydroxid, entsprechend 0,02 Gew.-% - bezogen auf die Einsatzstoffe - umgesetzt. Der Verlauf der Ringöffnungsreaktion wurde über die Änderung der Konzentration an Epoxidsauerstoff in der Reaktionsmischung verfolgt. Die Ergebnisse sind in Tab.1 zusammengefaßt.

**Tab.1:**

| Epoxidsauerstoffgehalte | | | | | | | | |
|---|---|---|---|---|---|---|---|---|
| Bsp. | Epoxidsauerstoffgehalt (Gew.-%) nach h | | | | | | | |
| | 0 | 1 | 2 | 3 | 4 | 5 | 7 | 10 |
| 1 | 1,3 | 1,3 | 0,8 | 0,4 | | 0,2 | | |
| 2 | 1,3 | 1,2 | | 0,6 | | 0,3 | 0,2 | |
| 3 | 2,0 | 1,5 | | 0,8 | | 0,3 | 0,1 | |
| 4 | 0,9 | 0,9 | | | | 0,5 | 0,3 | 0,1 |
| 5 | 1,9 | | 1,1 | | 0,6 | | 0,5 | 0,2 |
| V1 | 1,3 | 1,2 | | 0,6 | | 0,6 | | |

### Beispiel 6:

In einem 4-1-Stahlautoklaven wurden 2400 g (26 Mol) Glycerin vorgelegt, mit 0,45 g Lithiumhydroxid versetzt und auf 80°C erhitzt. Durch 5maliges Evakuieren und Belüften mit Stickstoff wurden anhaftende Wasserspuren entfernt. Danach wurde die Reaktionsmischung auf 150°C erhitzt und 1500 g (26 Mol) Propylenoxid portionsweise zudosiert, so daß der Druck im Reaktor einen Wert von 5 bar nicht überstieg. Nach Beendigung der Reaktion (ca. 3 h) wurde auf 80 bis 100°C abgekühlt und zur Entfernung von Spuren nichtumgesetzten Propylenoxids ca. 15 min evakuiert. Es wurden ca. 2900 g Glycerinpropoxylat in Form einer klaren, farblosen Flüssigkeit erhalten, die eine Hydroxylzahl von 1200 aufwies.

498 g (3,3 Mol) des lithiumhydroxid-haltigen Glycerinpropoxylates und 1495 g (1,7 Mol) Rüböl neuer Züchtung wurden unter Rühren bei 240°C über einen Zeitraum von 6 h einer Umesterung unterworfen. Es wurden ca. 1950 g einer klaren, leicht gelb gefärbten Flüssigkeit erhalten, die die folgenden Kennzahlen aufwies:

| | |
|---|---|
| Hydroxylzahl : | 280 |
| Verseifungszahl : | 142 |
| Säurezahl : | 0,8 |
| Li-Gehalt : | 7,5 ppm |

## Patentansprüche

1. Verfahren zur Herstellung von Epoxidringöffnungsprodukten, **dadurch gekennzeichnet**, daß man
a) Epoxidverbindungen mit Nucleophilen mit Ausnahme von Carbonsäuren in Gegenwart von 0,001 bis 0,01 Gew.% - bezogen auf die Einsatzstoffe - an Lithiumsalzen umsetzt und
b) die entstehenden Hydroxyverbindungen gegebenenfalls mit Fettsäureglyceridestern einer Umesterung unterwirft.

2. Verfahren nach Anspruch 1, **dadurch gekennzeichnet**, daß man als Epoxidverbindung Ethylenoxid, Propylenoxid und/ oder Butylenoxid einsetzt.

3. Verfahren nach Anspruch 1, **dadurch gekennzeichnet**, daß man als Epoxidverbindungen Epoxide von Olefinen der Formel **(I)** einsetzt,
R¹-CH=CH-R (I)
in der R¹ für einen linearen oder verzweigten aliphatischen Kohlenwasserstoffrest mit 1 bis 18 Kohlenstoffatomen und R für Wasserstoff oder einen linearen oder verzweigten Kohlenwasserstoffrest mit 1 bis 8 Kohlenstoffatomen steht.

4. Verfahren nach Anspruch 1, **dadurch gekennzeichnet**, daß man als Epoxidverbindungen Epoxide von Estern der Formel **(II)** einsetzt,
R³CO-OR⁴ (II)
in der R³CO für einen aliphatischen Acylrest mit 16 bis 24 Kohlenstoffatomen und 1 bis 5 Doppelbindungen und R⁴ für einen linearen oder verzweigten Alkylrest mit mit 1 bis 4 Kohlenstoffatomen steht.

5. Verfahren nach Anspruch 1, **dadurch gekennzeichnet**, daß man als Epoxidverbindungen Epoxide von Estern der Formel **(III)** einsetzt,
R⁵CO-OR⁶ (III)
in der R⁵CO für einen aliphatischen Acylrest mit 1 bis 24 Kohlenstoffatomen und 0 oder 1 bis 5 Doppelbindungen und R⁶ für einen linearen oder verzweigten aliphatischen Kohlenwasserstoffrest mit 16 bis 24 Kohlenstoffatomen und 1 bis 5 Doppelbindungen steht.

6. Verfahren nach Anspruch 1, **dadurch gekennzeichnet**, daß man als Epoxidverbindungen Epoxide von Fettsäureglycerinestern der Formel **(IV)** einsetzt, 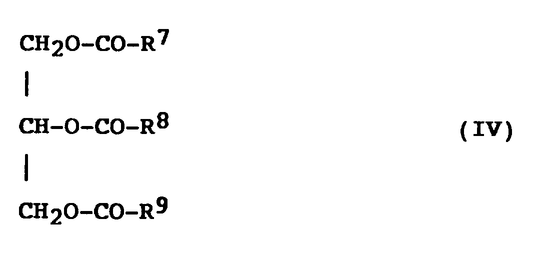 in der R⁷CO für einen linearen oder verzweigten aliphatischen Acylrest mit 16 bis 24 Kohlenstoffatomen und 1 bis 5 Doppelbindungen und R⁸CO und R⁹CO unabhängig voneinander für einen linearen oder verzweigten aliphatischen Acylrest mit 6 bis 24 Kohlenstoffatomen und 0 oder 1 bis 5 Doppelbindungen steht.

7. Verfahren nach mindestens einem der Ansprüche 1 bis 6, **dadurch gekennzeichnet**, daß man als Nucleophil Wasser einsetzt.

8. Verfahren nach mindestens einem der Ansprüche 1 bis 6, **dadurch gekennzeichnet**, daß man als Nucleophile Alkohole der Formel **(V)** einsetzt,
R¹⁰OH (V)
in der R¹⁰ für lineare oder verzweigte aliphatische Kohlenwasserstoffreste mit 1 bis 22 Kohlenstoffatomen und 0 oder 1 bis 3 Doppelbindungen steht.

9. Verfahren nach mindestens einem der Ansprüche 1 bis 6, **dadurch gekennzeichnet**, daß man als Nucleophile mehrwertige Alkohole einsetzt, ausgewählt aus der Gruppe, die von Ethylenglycol, Diethylenglycol, Polyethylenglycole im Molgewichtsbereich 300 bis 1500, Propandiol-1,2, Propandiol-1,3, Glycerin, Oligoglycerine mit Kondensationsgraden von durchschnittlich 2 bis 10, Trimethylolpropan, Pentaerythrit, Sorbitol und Sorbitan gebildet wird.

10. Verfahren nach mindestens einem der Ansprüche 1 bis 6, **dadurch gekennzeichnet**, daß man als Nucleophile Fettalkoholpolyglycolether der Formel **(VI)** einsetzt, 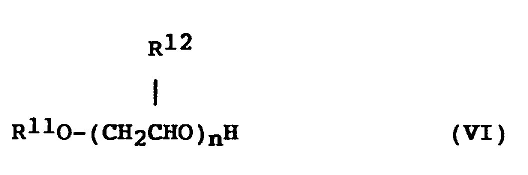 in der R¹¹ für lineare oder verzweigte aliphatische Kohlenwasserstoffreste mit 6 bis 22 Kohlenstoffatomen und 0 oder 1 bis 3 Doppelbindungen, R¹ für Wasserstoff oder eine Methylgruppe und n für Zahlen von 1 bis 30 steht.

11. Verfahren nach mindestens einem der Ansprüche 1 bis 10, **dadurch gekennzeichnet**, daß man die Epoxidverbindungen und die Nucleophile im molaren Verhältnis von 1 : 10 bis 10 : 1 einsetzt.

12. Verfahren nach mindestens einem der Ansprüche 1 bis 11, **dadurch gekennzeichnet**, daß man als Lithiumsalz Lithiumhydroxid einsetzt.

13. Verfahren nach mindestens einem der Ansprüche 1 bis 11, **dadurch gekennzeichnet**, daß man als Lithiumsalze Lithiumseifen von gegebenenfalls hydroxysubstituierten Fettsäuren mit 6 bis 22 Kohlenstoffatomen und 0, 1, 2 oder 3 Doppelbindungen einsetzt.

14. Verfahren nach mindestens einem der Ansprüche 1 bis 13, **dadurch gekennzeichnet**, daß man die Ringöffnung bei der Siedetemperatur des eingesetzten Nucleophils bzw. im Temperaturbereich von 100 bis 250°C durchführt.

15. Verfahren nach mindestens einem der Ansprüche 1 bis 14, **dadurch gekennzeichnet**, daß man als Fettsäureglyceridester Triglyceride der Formel **(VII)**, 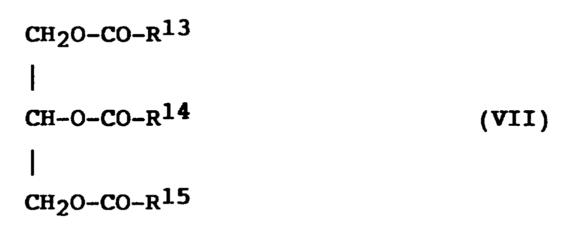 in die Umesterung einsetzt, in der R¹³CO für einen linearen oder verzweigten, gegebenenfalls hydroxy- und/oder alkoxysubstituierten aliphatischen Acylrest mit 16 bis 24 Kohlenstoffatomen und 1 bis 5 Doppelbindungen und R¹⁴CO und R¹⁵CO unabhängig voneinander für einen linearen oder verzweigten aliphatischen Acylrest mit 6 bis 24 Kohlenstoffatomen und 0 oder 1 bis 5 Doppelbindungen steht.

16. Verfahren nach mindestens einem der Ansprüche 1 bis 15, **dadurch gekennzeichnet**, daß man die Epoxide und die Fettsäureglyceridester im molaren Verhältnis von 10 : 1 bis 1 : 10 einsetzt.

17. Verfahren nach mindestens einem der Ansprüche 1 bis 16, **dadurch gekennzeichnet**, daß man die Umesterung im Temperaturbereich von 100 bis 250°C durchführt.

18. Verfahren nach mindestens einem der Ansprüche 1 bis 17, **dadurch gekennzeichnet**, daß man die Ringöffnung und die Umesterung in einem Schritt durchführt.

19. Epoxidringöffnungsprodukte, dadurch erhältlich, daß man
a) Epoxidverbindungen mit Nucleophilen mit Ausnahme von Carbonsäuren in Gegenwart von 0,001 bis 0,01 Gew.% - bezogen auf die Einsatzstoffe - an Lithiumsalzen umsetzt und
b) die entstehenden Hydroxyverbindungen gegebenenfalls mit Fettsäureglyceridestern einer Umesterung unterwirft.

20. Verwendung von Epoxidringöffnungsprodukten hergestellt nach dem Verfahren nach den Ansprüchen 1 bis 18 zur Herstellung von Alkydharzen und Polyurethanschäumen.

21. Verwendung von Epoxidringöffnungsprodukten nach Anspruch 19 zur Herstellung von Alkydharzen und Polyurethanschäumen.

## Claims

1. A process for the production of epoxide ring opening products, **characterized in that**
a) epoxide compounds are reacted with nucleophiles in the presence of 0.001 to 0.01% by weight - based on the starting materials - of lithium salts and
b) the hydroxy compounds formed are optionally transesterified with fatty acid glyceride esters.

2. A process as claimed in claim 1, **characterized in that** ethylene oxide, propylene oxide and/or butylene oxide is/are used as the epoxide compound.

3. A process as claimed in claim 1, **characterized in that** epoxides of olefins corresponding to formula (I):
R¹-CH=CH-R (I)
in which R¹ is a linear or branched aliphatic hydrocarbon radical containing 1 to 18 carbon atoms and R is hydrogen or a linear or branched hydrocarbon radical containing 1 to 8 carbon atoms,
are used as the epoxide compounds.

4. A process as claimed in claim 1, **characterized in that** epoxides of esters corresponding to formula (II):
R³CO-OR⁴ (II)
in which R³CO is an aliphatic acyl radical containing 16 to 24 carbon atoms and 1 to 5 double bonds and R⁴ is a linear or branched alkyl radical containing 1 to 4 carbon atoms,
are used as the epoxide compounds.

5. A process as claimed in claim 1 **characterized in that** epoxides of esters corresponding to formula **(III)**:
R⁵CO-OR⁶ (III)
in which R⁵CO is an aliphatic acyl radical containing 1 to 24 carbon atoms and 0 or 1 to 5 double bonds and R⁶ is a linear or branched aliphatic hydrocarbon radical containing 16 to 24 carbon atoms and 1 to 5 double bonds,
are used as the epoxide compounds.

6. A process as claimed in claim 1, **characterized in that** epoxides of fatty acid glycerol esters corresponding to formula **(IV)**: 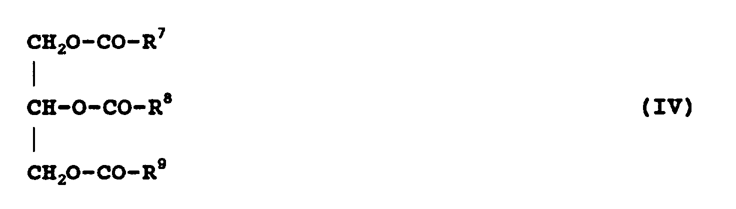 in which R⁷CO is a linear or branched aliphatic acyl radical containing 16 to 24 carbon atoms and 1 to 5 double bonds and R⁸CO and R⁹CO independently of one another represent a linear or branched aliphatic acyl radical containing 6 to 24 carbon atoms and 0 or 1 to 5 double bonds,
are used as the epoxide compounds.

7. A process as claimed in at least one of claims 1 to 6, **characterized in that** water is used as the nucleophile.

8. A process as claimed in at least one of claims 1 to 6, **characterized in that** alcohols corresponding to formula **(V)**:
R¹⁰OH (V)
in which R¹⁰ is a linear or branched aliphatic hydrocarbon radical containing 1 to 22 carbon atoms and 0 or 1 to 3 double bonds,
are used as the nucleophiles.

9. A process as claimed in at least one of claims 1 to 6, **characterized in that** polyhydric alcohols selected from the group consisting of ethylene glycol, diethylene glycol, polyethylene glycols with molecular weights in the range from 300 to 1,500, propane-1,2-diol, propane-1,3-diol, glycerol, oligoglycerols having degrees of condensation of on average 2 to 10, trimethylol propane, pentaerythritol, sorbitol and sorbitan, are used as the nucleophiles.

10. A process as claimed in at least one of claims 1 to 6, **characterized in that** fatty alcohol polyglycol ethers corresponding to formula (VI): 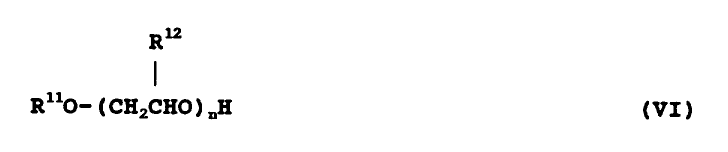 in which R¹¹ is a linear or branched aliphatic hydrocarbon radical containing 6 to 22 carbon atoms and 0 or 1 to 3 double bonds, R¹ is hydrogen or a methyl group and n is a number of 1 to 30,
are used as the nucleophiles.

11. A process as claimed in at least one of claims 1 to 10, **characterized in that** the epoxide compounds and the nucleophiles are used in a molar ratio of 1:10 to 10:1.

12. A process as claimed in at least one of claims 1 to 11, **characterized in that** lithium hydroxide is used as the lithium salt.

13. A process as claimed in at least one of claims 1 to 11, **characterized in that** lithium soaps of optionally hydroxy-substituted fatty acids containing 6 to 22 carbon atoms and 0, 1, 2 or 3 double bonds are used as the lithium salts.

14. A process as claimed in at least one of claims 1 to 13, **characterized in that** the ring opening reaction is carried out at the boiling temperature of the nucleophile used or at a temperature in the range from 100 to 250°C.

15. A process as claimed in at least one of claims 1 to 14, **characterized in that** triglycerides corresponding to formula **(VII)**: 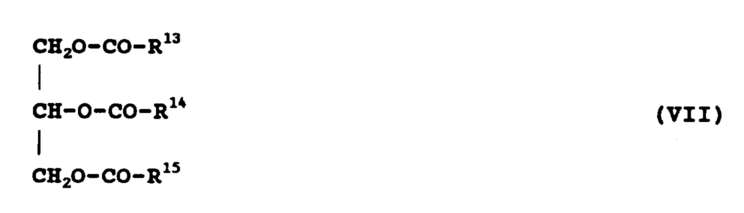 in which R¹³CO is a linear or branched, optionally hydroxy- and/or alkoxy-substituted aliphatic acyl radical containing 16 to 24 carbon atoms and 1 to 5 double bonds and R¹⁴CO and R¹⁵CO independently of one another represent a linear or branched aliphatic acyl radical containing 6 to 24 carbon atoms and 0 or 1 to 5 double bonds,
are used as the fatty acid glyceride esters.

16. A process as claimed in at least one of claims 1 to 15, **characterized in that** the epoxides and the fatty acid glyceride esters are used in a molar ratio of 10:1 to 1:10.

17. A process as claimed in at least one of claims 1 to 18, **characterized in that** the transesterification is carried out at a temperature in the range from 100 to 250°C.

18. A process as claimed in at least one of claims 1 to 17, **characterized in that** in that the ring opening and transesterification reactions are carried out in a single step.

19. Epoxide ring opening products obtainable by
a) reacting epoxide compounds with nucleophiles in the presence of 0.001 to 0.01% by weight - based on the starting materials - of lithium salts and
b) optionally subjecting the hydroxy compounds formed to transesterification with fatty acid glyceride esters.

20. The use of the epoxide ring opening products obtained by the process claimed in claims 1 to 18 for the production of alkyd resins and polyurethane foams.

21. The use of the epoxide ring opening products claimed in claim 19 for the production of alkyd resins and polyurethane foams.

## Revendications

1. Procédé de fabrication de produits d'ouverture du noyau époxy, **caractérisé en ce que** l'on
a) fait réagir des composés époxy avec des nucléophiles, à l'exception des acides carboxyliques, en présence de 0,001 à 0,01 % en poids (par rapport aux matières mises en oeuvre) de sels de lithium et
b) soumet à une transestérification les composés hydroxy formés, le cas échéant, avec des esters de glycérides d'acides gras.

2. Procédé selon la revendication 1, **caractérisé en ce que** l'on met en oeuvre comme composé époxy, de l'oxyde d'éthylène, de l'oxyde de propylène et/ou de l'oxyde de butylène.

3. Procédé selon la revendication 1, **caractérisé en ce que** l'on met en oeuvre comme composés époxy, des époxydes d'oléfines de la formule (I),
R¹-CH=CH-R (I)
dans laquelle R¹ représente un radical hydrocarbure aliphatique linéaire ou ramifié comportant 1 à 18 atomes de carbone et R correspond à l'hydrogène ou à un radical hydrocarbure linéaire ou ramifié possédant 1 à 8 atomes de carbone.

4. Procédé selon la revendication 1, **caractérisé en ce que** l'on met en oeuvre comme composés époxy, des époxydes d'esters de la formule (II),
R³CO-OR⁴ (II)
dans laquelle R³CO représente un radical acyle aliphatique comportant 16 à 24 atomes de carbone et 1 à 5 doubles liaisons, et R⁴ correspond à un radical alkyle linéaire ou ramifié possédant 1 à 4 atomes de carbone.

5. Procédé selon la revendication 1, **caractérisé en ce que** l'on met en oeuvre comme composés époxy, des époxydes d'esters de la formule (III)
R⁵CO-OR⁶ (III)
dans laquelle R⁵CO représente un radical acyle aliphatique comportant 1 à 24 atomes de carbone et 0 ou 1 à 5 doubles liaisons, et R⁶ correspond à un radical hydrocarbure aliphatique linéaire ou ramifié comportant 16 à 24 atomes de carbone et 1 à 5 doubles liaisons.

6. Procédé selon la revendication 1, **caractérisé en ce que** l'on met en oeuvre comme composés époxy, des époxydes d'esters de glycérine d'acides gras de la formule (IV), 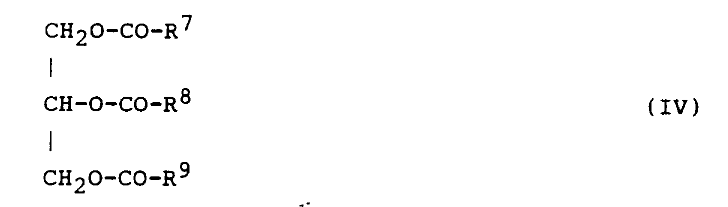 dans laquelle R⁷CO représente un radical acyle aliphatique linéaire ou ramifié comportant 16 à 24 atomes de carbone et 1 à 5 doubles liaisons et R⁸CO et R⁹CO, correspondent indépendamment l'un de l'autre, à un radical acyle aliphatique linéaire ou ramifié comportant 6 à 24 atomes de carbone et 0 ou 1 à 5 doubles liaisons.

7. Procédé selon au moins une des revendications 1 à 6, **caractérisé en ce que** l'on utilise comme nucléophile, de l'eau.

8. Procédé selon au moins une des revendications 1 à 6, **caractérisé en ce que** l'on utilise comme nucléophiles, des alcools de la formule (V),
R¹⁰OH (V)
dans laquelle R¹⁰ représente un radical hydrocarbure aliphatique linéaire ou ramifié comportant 1 à 22 atomes de carbone et 0 ou 1 à 3 doubles liaisons.

9. Procédé selon au moins une des revendications 1 à 6, **caractérisé en ce que** l'on utilise comme nucléophiles, des alcools polyvalents, sélectionnés parmi le groupe formé des composés suivants: éthylèneglycol, diéthylèneglycol, polyéthylèneglycols dans la plage de poids moléculaire de 300 à 1500, propanediol-1,2, propanediol-1,3, glycérine, oligoglycérines présentant des degrés de condensation de 2 à 10 en moyenne, triméthylolpropane, pentaérythrite, sorbitol et sorbitane.

10. Procédé selon au moins une des revendications 1 à 6, **caractérisé en ce que** l'on utilise comme nucléophiles, des polyglycoléthers d'alcools gras de la formule (VI), 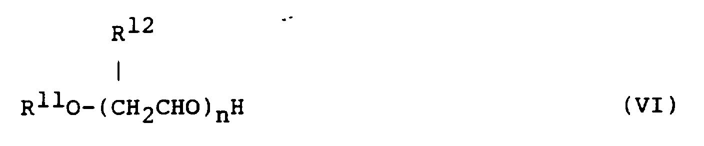 dans laquelle R¹¹ représente un radical hydrocarbure aliphatique linéaire ou ramifié comportant 6 à 22 atomes de carbone et 0 ou 1 à 3 doubles liaisons, R¹ correspond à l'hydrogène ou à un groupe méthyle et n représente un nombre de 1 à 30.

11. Procédé selon au moins une des revendications 1 à 10, **caractérisé en ce que** l'on met en oeuvre les composés époxy et les nucléophiles en rapport molaire de 1:10 à 10:1.

12. Procédé selon au moins une des revendications 1 à 11, **caractérisé en ce que** l'on met en oeuvre comme sel de lithium, de l'hydroxyde de lithium.

13. Procédé selon au moins une des revendications 1 à 11, **caractérisé en ce que** l'on met en oeuvre comme sels de lithium, des savons de lithium d'acides gras, le cas échéant, à substitution hydroxyle, comportant 6 à 22 atomes de carbone et 0, 1, 2 ou 3 doubles liaisons.

14. Procédé selon au moins une des revendications 1 à 13, **caractérisé en ce que** l'on opère l'ouverture du noyau à la température d'ébullition du nucléophile mis en oeuvre ou dans la plage de températures de 100 à 250 °C.

15. Procédé selon au moins une des revendications 1 à 14, **caractérisé en ce que** l'on met en oeuvre comme esters de glycérides d'acides gras dans la transestérification, des triglycérides de la formule (VII), 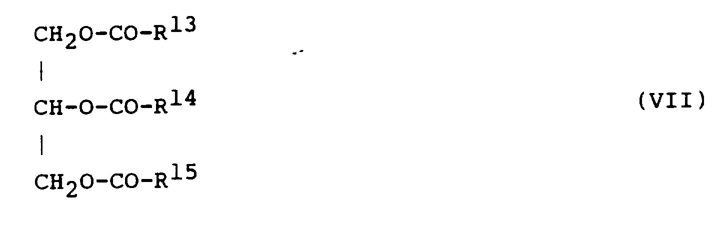 dans laquelle R¹³CO représente un radical acyle aliphatique linéaire ou ramifié, le cas échéant à substitution hydroxyle et/ou alcoxyle, comportant 16 à 24 atomes de carbone et 1 à 5 doubles liaisons, et R¹⁴CO et R¹⁵CO correspondent indépendamment l'un de l'autre à un radical acyle aliphatique linéaire ou ramifié comportant 6 à 24 atomes de carbone et 0 ou 1 à 5 doubles liaisons.

16. Procédé selon au moins une des revendications 1 à 15, **caractérisé en ce que** l'on met en oeuvre les époxydes et les esters de glycérides d'acides gras dans un rapport molaire de 10:1 à 1:10.

17. Procédé selon au moins une des revendications 1 à 16, **caractérisé en ce que** l'on opère la transestérification dans la plage de températures de 100 à 250 °C.

18. Procédé selon au moins une des revendications 1 à 17, **caractérisé en ce que** l'on opère l'ouverture du noyau et la transestérification en une seule étape.

19. Produits d'ouverture du noyau époxy, obtenables en
a) faisant réagir des composés époxy avec des nucléophiles, à l'exception des acides carboxyliques, en présence de 0,001 à 0,01 % en poids (par rapport aux matières mises en oeuvre) de sels de lithium et
b) en soumettant à une transestérification les composés hydroxy formés, le cas échéant, avec des esters de glycérides d'acides gras.

20. Utilisation des produits d'ouverture du noyau époxy fabriqués selon le procédé conforme aux revendications 1 à 18 pour la fabrication de résines alkydes et de mousses de polyuréthane.

21. Utilisation des produits d'ouverture du noyau époxy selon la revendication 19, pour la fabrication de résines alkydes et de mousses de polyuréthane.
